(19) Europäisches Patentamt
European Patent Office
Office européen des brevets

(11) EP 3 591 400 B1

(12) # EUROPÄISCHE PATENTSCHRIFT

(45) Veröffentlichungstag und Bekanntmachung des Hinweises auf die Patenterteilung:
**01.03.2023 Patentblatt 2023/09**

(21) Anmeldenummer: **18181192.8**

(22) Anmeldetag: **02.07.2018**

(51) Internationale Patentklassifikation (IPC):
***G01N 33/543*** *(2006.01)* ***G01N 1/30*** *(2006.01)*

(52) Gemeinsame Patentklassifikation (CPC):
**G01N 33/543; G01N 1/2813**

(54) **BESCHICHTETE FESTPHASENFRAGMENTE UND DEREN HERSTELLUNG**

COATED SOLID-PHASE FRAGMENTS AND ITS PRODUCTION

FRAGMENTS DE PHASE SOLIDE REVÊTUS ET LEUR FABRICATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**08.01.2020 Patentblatt 2020/02**

(73) Patentinhaber: **Euroimmun Medizinische Labordiagnostika AG**
**23560 Lübeck (DE)**

(72) Erfinder:
- **Eggert-Gospos, Dirk**
  **23847 Schürensöhlen (DE)**
- **Rateike, Martin**
  **23689 Pansdorf (DE)**
- **Stöcker, Winfried**
  **23627 Groß Grönau (DE)**
- **Rottmann, Norbert**
  **23570 Lübeck (DE)**

(56) Entgegenhaltungen:
**WO-A1-2005/073693**
**WO-A1-2007/140889**
**WO-A1-2015/125343**
**US-A- 4 647 543**
**US-A1- 2014 255 977**
**US-A1- 2014 255 978**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

Die vorliegende Erfindung richtet sich auf ein Verfahren zur Herstellung von mit biologischem Material beschichteten Festphasenfragmenten.

Viele Testsysteme für die klinische und medizinische Diagnostik, Veterinärmedizin, pharmazeutische und toxikologische Untersuchungen, Lebensmittel- und Umweltanalytik sowie für allgemein-biologische und biochemische Analysen basieren auf dem Einsatz Festphasen-gebundener Bioreagenzien (Festphasen-Tests). Ein klassisches Beispiel sind immunologische Testsysteme (Immunoassays), ohne die zahlreiche Bereiche der modernen Diagnostik und Analytik heutzutage nicht mehr vorstellbar sind. Ein Immunoassay ist eine Technik, bei der mittels einer immunologischen Reaktion die Anwesenheit einer Substanz gemessen wird. Charakteristisch für jede immunologische Reaktion ist eine Wechselwirkung zwischen Antikörper und Antigen. Das Resultat dieser Wechselwirkung ist ein Antikörper-Antigen-Komplex. Durch *in vitro* Assays können der Antikörper oder das korrespondierende Antigen nachgewiesen werden. Der Nachweis des Antikörpers setzt die Zugabe des entsprechenden Antigens zum Testsystem voraus und umgekehrt. Antikörper (Immunglobuline) sind eine besondere Kategorie von Proteinen. Antigene können biologische oder organische Moleküle, aber auch andere chemische Verbindungen sein. Die meisten Immunoassays verlangen eine Trennung von gebundenem und freiem Antikörper und Antigen (heterogene Immunoassays oder Festphasen-Immunoassays). Diese Trennung wird dadurch bewerkstelligt, dass ein Reaktionspartner (Antikörper oder Antigen) an eine Festphase gebunden wird. Beispielsweise können nicht-gebundene Antigene einfach von Antikörper gebundenen Antigenen separiert werden, wenn der Antikörper an eine Festphase gekoppelt ist. Wird der Antikörper oder das Antigen in Form eines Biomoleküls (z.B. Proteins, Nukleinsäure, Polysaccharids, Lectins) oder als Bestandteil einer biologischen Substanz (Gewebeschnitt, Mikroorganismus, Zelle) an die feste Phase gebunden, spricht man von einem Festphasen-gebundenen Bioreagenz.

Festphasen-Tests sind im Allgemeinen leichter auszuführen, sensitiver und einfacher zu automatisieren als Flüssigphasen-Tests, bei denen sich die Reaktionspartner als auch die Reaktionsprodukte in Lösung befinden.

Eine besondere Form der diagnostischen Festphasen-Tests stellt der sogenannte indirekte Immunfluoreszenztest (IIFT) dar. Um beispielsweise Autoantikörper oder Infektionsantikörper in einer Patientenprobe zu identifizieren, werden Zellen, Gewebeschnitte oder aufgereinigte, biochemisch charakterisierte Substanzen als Antigen-Substrate auf einem Mikroskop-Objektträger eingesetzt. In einem ersten Inkubationsschritt binden sich bei positiven Proben die nachzuweisenden Antikörper aus verdünntem Patientenserum an die Festphasen-gebundenen Antigene. In einem zweiten Inkubationsschritt werden diese Antikörper mit Fluoreszenz-markierten Anti-Human-Antikörpern sichtbar gemacht. Im Fluoreszenzmikroskop werden die gebundenen Antikörper und ggf. deren spezifische Lokalisation innerhalb der Substrat-Zelle oder des Substrat-Gewebes identifiziert.

Indirekte Immunfluoreszenztests ermöglichen eine hohe Spezifität, d.h. positive und negative Proben ergeben einen großen Signalunterschied. Des Weiteren zeigt sich für jeden gebundenen Antikörper ein typisches Fluoreszenzmuster, je nach Lokalisation der einzelnen Antigene, was zur Identifizierung der Autoantikörper des Patienten genutzt werden kann. Ferner steht das gesamte Antigenspektrum der Ausgangssubstrate zur Verfügung, so dass viele Antikörper erfasst werden und eine hohe Trefferquote erzielt werden kann. Außerdem ist der indirekte Immunfluoreszenztest die Methode der Wahl, wenn es nicht möglich ist, Testantigene analysegerecht für Enzymimmuntests aufzubereiten.

Die Firma EUROIMMUN (Lübeck, Deutschland) hat ein besonderes Herstellungsverfahren und besonders bestückte Objektträger entwickelt, um IIFTs effizient durchzuführen. Es werden Objektträger angeboten, die mit mehreren mit biologischem Material beschichteten Deckgläsern ("BIOCHIP") bestückt sind. Die BIOCHIP-Technologie umfasst einen Aktivierungsschritt, bei dem Kulturzellen oder Gewebeschnitte auf physikalisch oder chemisch aktivierte Deckgläser aufgebracht werden. Ferner können Gefrierschnitte kovalent auf der Glasoberfläche verankert werden. Die starke Hafthaftung zwischen Substrat und Deckglas ermöglicht es, die Deckgläser mit dem biologischen Material maschinell in millimetergroße Fragmente (BIOCHIPs) zu unterteilen. So lassen sich pro Gewebeschnitt 10 und mehr Präparate von einheitlicher Qualität gewinnen.

In EP 0117262 B1 werden die oben genannten BIOCHIPs offenbart.

US 4647543 A offenbart ein Verfahren zur Probenzubereitung für immunohistochemische Analyse.

WO 2005/073693 A1 offenbart ein Verfahren zur Herstellung Festphasen-gebundener Bioreagenzien als Bestandteil von Vorrichtungen für Untersuchungen an unbeweglich gemachtem biologischem Material.

WO 2007/140889 A1 offenbart ein Verfahren bei dem mit Gewebe beschichtete Festphasenträger fragmentiert und anschließend kombiniert in Form eines Trägers zusammengesetzt werden.

US2014/255978 A1 offenbart ein Verfahren zur Herstellung von mit biologischem Material beschichteten Festphasenfragmenten der vorliegenden Anmeldung, wobei auf einem Glassubstrat Zellen aufgebracht sind. Auf der gegenüberliegenden Seite wird eine haftende Folie angebracht, die dehnbar ist. Das Glassubstrat hat Sollbruchstellen. Durch anlegenden einer Zugspannung wird das Glassubstrat in Stücke zerlegt. Diese werden anschließend von der Folie mit Hilfe einer Nadel abgelöst.

In EP 1718948 B1 wird der oben genannte IIFT und ein entsprechendes Herstellungsverfahren von BIOCHIPs beschrieben. Bei diesem Herstellungsverfahren wird eine Nadel verwendet, die eine Klebefolie durchsticht, auf der sich mit biologischem Material beschichtete Deckglasfragmente befinden. Die Nadel durchsticht die Folie so, dass die Deckglasfragmente durch einen Saugnapf von der Folie entnommen werden können. Mit diesem Herstellungsverfahren können allerdings keine größeren (größer als 3,25 mm Kantenlänge) BIOCHIPs hergestellt werden.

Für unterschiedliche Anwendungen, z.B. pathologische Untersuchungen von Patientengewebe oder die gleichzeitige Untersuchung mehrerer verschiedener Gewebearten, wäre es wünschenswert auch größere BIOCHIPs herstellen zu können. Daher besteht ein Bedarf an einem Verfahren, das eine reproduzierbare Herstellung von größeren mit biologischem Material beschichteten Deckgläsern erlaubt, sowie an derartigen beschichteten Deckgläsern.

Herstellungsverfahren werden nachfolgend beschrieben und sind Gegenstand der vorliegenden Erfindung.

Die Erfinder der vorliegenden Erfindung haben überraschenderweise gefunden, dass die Verwendung einer Nadel im Ablöseschritt von Herstellungsverfahren von mit biologischem Material beschichteten Festphasenfragmenten durch einen Schritt des Vorlösens mit einem Hebekopf ersetzt werden kann. Durch diese Änderung ist die Herstellung wesentlich größerer Festphasenfragmente/Deckglasfragmente möglich. Im Detail wird im erfindungsgemäßen Verfahren an einer Folie, die mit Festphasenfragmenten beschichtet ist, eine Zugspannung erzeugt. Anschließend wird die Folie mit einem Hebekopf kontaktiert, um ein oder mehrere Festphasenfragmente von der Folie vorzulösen.

Die Erfindung richtet sich daher auf ein Verfahren zur Herstellung von mit biologischem Material beschichteten Festphasenfragmenten, umfassend:

- Bereitstellen eines mit biologischem Material beschichteten Festphasenträgers, wobei der Festphasenträger fragmentiert ist und die Fragmente an einer Folie haften;
- Erzeugen einer Zugspannung an der mit den Festphasenfragmenten beschichteten Folie;
- Kontaktieren der Folie mit einem Hebekopf, um ein Festphasenfragment von der Folie in der Weise vorzulösen, dass mindestens 20% einer Oberflächenseite des Festphasenfragments von der Folie abgelöst werden, wobei im Kontaktierungsschritt der Hebekopf die Folie in Richtung der beschichteten Oberfläche des Festphasenfragments drückt und wobei das Drücken durch den Hebekopf die Folie nicht beschädigt, zerreißt oder zersticht; und
- finales Ablösen des einen vorgelösten Festphasenfragmentes von der Folie mittels einer Einheit mit einem Saugnapf, so dass 100% der zuvor gebundenen Oberflächenseite des Festphasenfragmentes von der Folie abgelöst sind.

Bevorzugte Ausführungsformen des erfindungsgemäßen Verfahrens werden nachfolgend beschrieben, wobei (a) die mit biologischem Material beschichtete Oberfläche (2) des Festphasenfragments mindestens 10,25 $mm^2$, bevorzugt mindestens 25 $mm^2$ umfasst; (b) die Länge mindestens einer Kante (4, 5) des Festphasenfragments größer als 3,25 mm, bevorzugt größer als 5 mm ist; und/oder (c) das Festphasenfragment ein Glasfragment ist.

In bevorzugten Ausführungsformen ist die Festphasenfragmentdicke (3) zwischen 0,05 mm und 0,3 mm, bevorzugt zwischen 0,1 mm und 0,2 mm.

In weiteren bevorzugten Ausführungsformen ist das biologische Material über ein Linkermolekül an den Festphasenträger gebunden. Das Linkermolekül kann (a) eine Silicium umfassende Verbindung, bevorzugt ein Silylether sein; oder (b) eine Struktur ausgewählt aus der Gruppe bestehend aus

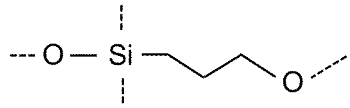

und

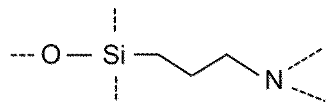

umfassen.

In bevorzugten Ausführungsformen des erfindungsgemäßen Verfahrens ist mindestens 70%, bevorzugt mindestens 85% der Oberfläche (2) des Festphasenfragments mit biologischem Material beschichtet.

In weiteren bevorzugten Ausführungsformen wird die Zugspannung dadurch erzeugt, dass die Folie über einen, bevorzugt kreisförmigen Rahmen gespannt ist.

In weiteren bevorzugten Ausführungsformen des erfindungsgemäßen Verfahrens ist die Folie eine Klebefolie, die (a) Polyvinylchlorid (PVC); und/oder (b) Acrylkleber umfasst.

Offenbart wird ferner ein mit biologischem Material beschichtetes Festphasenfragment, erhältlich durch das erfindungsgemäße Verfahren.

Offenbart wird ferner ein Träger, auf dem mindestens ein erfindungsgemäßes Festphasenfragment aufgebracht ist.

Offenbart wird ferner eine Vorrichtung zur Herstellung von mit biologischem Material beschichteten Festphasenfragmenten, umfassend:

- Zugspannungseinheit ausgebildet zum Erzeugen von Zugspannung an einer Folie;
- Hebekopfeinheit ausgebildet zum Vorlösen von Festphasenfragmenten von einer Folie; und

- Entnahmeeinheit ausgebildet zum Abnehmen von Festphasenfragmenten von einer Folie.

Die Vorrichtung kann ferner mindestens eine Steuereinheit umfassen.

Offenbart wird ferner ein Festphasenfragment, das mit biologischen Material beschichtet ist und wobei (a) die mit biologischem Material beschichtete Oberfläche (2) des Festphasenfragments mindestens 10,25 $mm^2$, bevorzugt mindestens 25 $mm^2$ umfasst; und/oder (b) die Länge mindestens einer Kante (4, 5) des Festphasenfragments größer als 3,25 mm, bevorzugt größer als 5 mm ist. Bevorzugt ist das Festphasenfragment ein Glasfragment. Die Festphasenfragmentdicke (3) kann zwischen 0,05 mm und 0,3 mm, bevorzugt zwischen 0,1 mm und 0,2 mm betragen. Das biologische Material kann über ein Linkermolekül an den Festphasenträger gebunden sein. Das Linkermolekül kann (a) eine Silicium umfassende Verbindung, bevorzugt ein Silylether sein; oder (b) eine Struktur ausgewählt aus der Gruppe bestehend aus

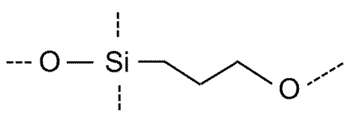

und

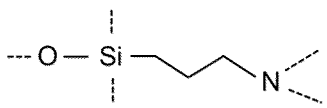

umfassen. Das Festphasenfragment ist zu mindestens 70%, bevorzugt mindestens 85% einer Oberfläche (2) mit biologischem Material beschichtet.

Der Ausdruck "biologisches Material", wie hierin verwendet, bezieht sich auf Material, das aus lebenden Organismen isoliert oder abgeleitet wird. Beispiele für biologisches Material umfassen, ohne darauf beschränkt zu sein, Proteine, Nukleinsäuren, Lipide, Zellen, Gewebe, Gewebeschnitte, Organe, Organschnitte, zellbasierte Konstrukte oder Kombinationen davon. In einigen Aspekten kann sich biologisches Material auf Säugetierzellen beziehen. In anderen Aspekten kann sich biologisches Material auf immortalisierte Zellkulturen, Blutplättchen, Bakterien, Viren, Säugetierzellmembranen, Liposomen, Enzyme oder Kombinationen davon beziehen. In anderen Aspekten kann sich biologisches Material auf Fortpflanzungszellen beziehen, einschließlich Samenzellen, Spermatozyten, Oozyten, Eizellen, Embryonen, Keimbläschen oder Kombinationen davon. In anderen Aspekten kann sich biologisches Material auf Vollblut, Serum, rote Blutkörperchen, weiße Blutzellen, Blutplättchen, Zerebrospinalflüssigkeit, Viren, Bakterien, Algen, Pilze oder Kombinationen davon beziehen. Biologisches Material umfasst auch rekombinant hergestellte und ggf. aufgereinigte Proteine und Antikörper, wie eine homogene Population monoklonaler oder eine heterogene Population polyklonaler Antikörper.

Der Ausdruck "Beschichtung", wie er in dieser Anmeldung verwendet wird, soll sich auf das Aufbringen von biologischem Material auf die Oberfläche eines Festphasenfragments beziehen. Die Beschichtung umfasst die Bindung durch Adhäsionskräfte, die Bindung durch ein Linkermolekül, Filmbeschichtung und Inkrustierung. Die Beschichtung kann vollständig oder teilweise sein, beispielsweise über 30% oder mehr, über 40% oder mehr, über 50% oder mehr, über 60% oder mehr, über 70% oder mehr, über 75% oder mehr, über 80% oder mehr, über 85% oder mehr, über 90% oder mehr, über 95% oder mehr, über 97% oder mehr der Oberfläche des Festphasenfragments.

Der Ausdruck "Linkermolekül" oder "Linker", wie hierin austauschbar verwendet, bezieht sich auf jede molekulare Einheit, die eine Bindungsanlagerung mit einem Abstand zwischen zwei oder mehr Molekülen, Molekülgruppen und/oder Moleküleinheiten vermittelt. Bevorzugt besitzt das Linkermolekül eine kovalente Bindung an das Festphasenfragment und eine weitere kovalente Bindung an das biologische Material. Beispielhafte Linker, die in Festphasenfragmenten und Verfahren dervorliegenden Erfindung verwendet werden können, sind Polymere, beispielsweise Polyethylenglycol (PEG)-Einheiten, wobei die Polymerketten weiterhin Alkyl-, Alken-, Alkin-, Ester-, Ether-, Amid-, Imid-, Sulfo- und/oder Phosphodiestergruppen umfassen können. Besonders bevorzugt ist das Linkermolekül eine Silicium (Si) umfassende Verbindung, noch weiter bevorzugt ein Silylether der Grundstruktur $R_1R_2R_3Si\text{-}O\text{-}R_4$, wobei $R_1$, $R_2$ und $R_3$ nicht-substituierte oder substituierte organische Gruppen und $R_4$ eine Alkyl- oder Arylgruppe darstellen.

"Festphasenfragment", wie hierin offenbart, bezieht sich auf jedes Festphasenmaterial, an das ein Biomolekül angeheftet werden kann. Beispielhafte feste Substrate, die mit biologischen Material und Verfahren der vorliegenden Offenbarung verwendet werden können, umfassen Kügelchen, Objektträger, Vertiefungen, Chips, die aus verschiedenen Festphasenmaterialien einschließlich Glas, Polymer und Silizium hergestellt werden können. Besonders bevorzugt ist das Festphasenfragment ein mit biologischem Material beschichteter Glasträger. Der Ausdruck "Festphasenträger", wie hierin verwendet, bezeichnet ein festes beschichtetes Substrat wie oben beschrieben, jedoch vor seiner Fragmentierung. Insofern treffen die oben genannten Materialeigenschaften eines Festphasenfragments auch auf einen Festphasenträger zu.

Der Ausdruck "fragmentiert", wie hierin verwendet, richtet sich auf Festphasenträger, die in zwei oder mehr Teilstücke (Fragmente) verkleinert werden. Dem Fachmann sind diverse Techniken zum Fragmentieren, insbesondere von beschichteten Glasträgern, bekannt. Diese umfassen, sind aber nicht beschränkt auf Brechen,

Schneiden (durch ein Schneidegerät mit Klinge oder Wasserstrahl), Lasern und Ritzen. Die Form der sich ergebenden Teilstücke kann einheitlich oder divers sein. In bevorzugten Ausführungsformen sind die Teilstücke einheitlich geformt, insbesondere als Rechtecke. In noch weiter bevorzugten Ausführungsformen sind die Teilstücke Quadrate. Bevorzugt besitzen die fragmentierten Teilstücke mindestens eine Oberfläche, die nicht größer als 1500 $mm^2$, nicht größer als 1000 $mm^2$, nicht größer als 750 $mm^2$, nicht größer als 500 $mm^2$, nicht größer als 400 $mm^2$, nicht größer als 300 $mm^2$, nicht größer als 200 $mm^2$ oder nicht größer als 120 $mm^2$ ist. In anderen bevorzugten Ausführungsformen ist die Kantenlänge (4, 5) mindestens einer Seite nicht länger als 150 $mm^2$, nicht länger als 130 $mm^2$, nicht länger als 100 $mm^2$, nicht länger als 80 $mm^2$, nicht länger als 50 $mm^2$, nicht länger als 40 $mm^2$, nicht länger als 30 $mm^2$ oder nicht länger als 25 $mm^2$.

Der Ausdruck "Folie", wie hierin verwendet, bezieht sich auf eine Adhäsionsfolie oder eine Klebefolie. Eine Adhäsionsfolie im Sinne der vorliegenden Erfindung ist eine Folie, die durch Adhäsionskräfte an ihrer Oberfläche eine Haftung an Glas, bevorzugt mit einer Dicke zwischen 0,001 mm und 1,5 mm, ermöglicht. In bevorzugten Ausführungsformen ist die Adhäsionsarbeit, d.h. die Kraft, die aufgewendet werden muss um die Adhäsionsfolie und das Festphasenfragment zu trennen, mindestens 0,00001 J/$mm^2$, mindestens 0,00001 J/$mm^2$, mindestens 0,00001 J/$mm^2$, mindestens 0,00001 J/$mm^2$, mindestens 0,0001 J/$mm^2$, mindestens 0,001 J/$mm^2$, mindestens 0,01 J/$mm^2$, mindestens 0,1 J/$mm^2$, mindestens 1 J/$mm^2$, mindestens 10 J/$mm^2$ oder mindestens 100 J/$mm^2$.

Unter "Klebefolie", wie hierin verwendet, wird eine einseitig oder beidseitig mit einem Klebstoff versehene (Kunststofffolie verstanden. Ein Klebstoff ist natürliches oder synthetisches Material, das an der Stelle der topischen Anwendung haften kann. Bevorzugt kann der Klebstoff an Glas haften. Weiter bevorzugt sind geeignete Klebstoffe ausgewählt aus Polyisobutylen, Acrylate, Silicone, Polyisoalkylene, Polyetherblockamidcopolymere, Polybutadien, Styrol-Butadien (oder Isopren)-Styrol-Blockcopolymer-Kautschuk, auf Vinyl basierende hochmolekulare Materialien wie z.B. Polyvinylalkylether, Polyvinylacetat, Ethylenvinylacetat-Copolymere, ein teilweise verseifte Produkte von Polyvinylacetat, Polyvinylalkohol und Polyvinylpyrrolidon, Polyurethan oder eine Kombination davon. In bevorzugten Ausführungsformen ist der Klebstoff ein Acrylkleber. Die Acrylklebstoffe umfassen vernetzte und unvernetzte Acrylcopolymere, ausgewählt aus Polymethacrylat, wie Butylacrylat, Ethylhexylacrylat, Vinylacetat, (Meth)acrylsäure, wie Butyl(meth)acrylat, Pentyl(meth)acrylat, Hexyl(meth)acrylat, Heptyl(meth)acrylat, Octyl(meth)acrylat, Nonyl(meth)acrylat, Decyl(meth)acrylat, Undecyl(meth)acrylat, Dodecyl(meth)acrylat und Tridecyl(meth)acrylat und Copolymere von mindestens einem der obigen Ester und andere damit copolymerisierbare Monomere. Bevorzugte Acrylatpolymere sind im Handel unter dem Handelsnamen Gelva®, z.B. Gelva® 3011 oder DuroTak®, erhältlich von National Starch and Chemical Company, und Zutphen, Holland, z.B. DuroTak® 202A, DuroTak® 608, DuroTak® 4201, DuroTak® 2510, DuroTak® 8710, DuroTak® 87-2353, DuroTak® 87-2353, DuroTak® 87-2353, DuroTak® 87-2353 ® 387-2051 oder DuroTak® 387-2052. In weiter bevorzugten Ausführungsformen ist die Klebefolie die Folie 1009R (Silicone-free blue) oder die Folie 1020R (UV curable adhesive film (PVC)) jeweils hergestellt und vertrieben von Ultron Systems (Moorpark, USA).

Das Folienmaterial (von Adhäsions- und Klebefolie) kann ein dünnes flexibles Folienmaterial sein und umfasst Polymerfilme, wie Polyvinylchlorid (PVC), Polyethylen (PE), Polypropylen (PP), Polyethylenterephthalat (PET), Polystyrol (PS), Polyamid (PA), vergleichbare Polymere und Kombinationen davon; und Metallfolien, wie Aluminium (Al). Bevorzugt wird eine Polyvinylchlorid (PVC) Folie verwendet.

Der Ausdruck "Zugspannung" oder "mechanische Spannung", wie hierin austauschbar verwendet, wird durch das Symbol "σ" dargestellt und ist auf einer gedachten Schnittfläche durch einen Körper die Kraftkomponente $F_i$ in i-Richtung bezogen auf die Fläche A (Normale n), auf der sie wirkt. Sie kann wie folgt berechnet werden:

$$\sigma_{ni} = \lim_{\Delta A \to 0} \frac{\Delta F_i}{\Delta A}$$

In bevorzugten Ausführungsformen beträgt die Zugspannung an der mit Festphasenfragmenten bestückten Folie mindestens 0,00001 N/$mm^2$, mindestens 0,00001 N/$mm^2$, mindestens 0,00001 N/$mm^2$, mindestens 0,00001 N/$mm^2$, mindestens 0,0001 N/$mm^2$, mindestens 0,001 N/$mm^2$, mindestens 0,01 N/$mm^2$, mindestens 0,1 N/$mm^2$, mindestens 1 N/$mm^2$, mindestens 10 N/$mm^2$ oder mindestens 100 N/$mm^2$.

"Hebekopf", wie hierin verwendet, bezieht sich auf ein Arbeitsmittel, das mit einer mit Festphasenfragmenten bestückten Folie in Kontakt gebracht werden kann und das so ausgebildet ist, dass mit dessen Hilfe die Fragmente von der Folie durch das erfindungsgemäße Verfahren entnommen werden können. Insofern besitzt der Hebekopf mindestens eine Seite/Fläche, mit der die Folie kontaktiert werden kann. Diese Seite/Fläche des Hebekopfs kann jegliche Formen haben, ist aber bevorzugt rund oder oval. In anderen bevorzugten Ausführungsformen hat diese Fläche/Seite des Hebekopfs einen Umfang oder Durchmesser von 0,001 mm bis 6 mm, von 0,05 mm bis 5 mm, von 0,1 mm bis 4 mm, von 0,5 mm bis 3 mm, von 0,7 mm bis 2 mm oder von 1 mm bis 1,5 mm. In weiteren bevorzugten Ausführungsformen besitzt die Hebekopfeinheit mindestens einen Hebekopf, der wie oben beschrieben ausgebildet ist.

Der Ausdruck "vorlösen", wie hierin verwendet, bezieht sich auf das partielle Ablösen eines Festphasenfragments von der Folie. In bevorzugten Ausführungsformen bezeichnet "vorlösen" das Ablösen von mindestens 20%, mindestens 30%, mindestens 35%, mindestens 40%, mindestens 45%, mindestens 50%, mindestens 55%, mindestens 60%, mindestens 65%, mindestens 70%, mindestens 75%, mindestens 80%, mindestens 85%, mindestens 90%, mindestens 93% oder mindestens 97% einer Oberflächenseite des Festphasenfragments von der Folie. Ohne an eine bestimmte Theorie gebunden sein, wird vermutet, dass das Vorlösen im erfindungsgemäßen Verfahren durch das Zusammenwirken der Zugspannung auf die Folie und das Kontaktieren und Drücken des Hebekopfs in Richtung der beschichteten Oberfläche entsteht. Durch eine derartige Bewegung des Hebekopfs unter dem Festphasenfragment scheint die Folie ausgelenkt zu werden und sich von den Bereichen des Fragments zu lösen, die nicht durch den Hebekopf berührt werden (siehe Figur 5).

Der Ausdruck "abnehmen" oder "entnehmen", wie hierin austauschbar verwendet, bezieht sich auf das finale Ablösen des Festphasenfragments von der Folie. D.h. 100% zuvor gebundenen Oberflächenseite des Festphasenfragments sind von der Folie abgelöst. Nach dem Abnehmen besteht zwischen dem Festphasenfragment und der Folie keine direkte oder indirekte Verbindung. Ein solcher Schritt wird über eine Einheit mit einem Saugnapf ausgeführt.

Eine mit biologischem Material beschichtete Oberfläche des Festphasenfragments ist in Figur 1 unter dem Bezugszeichen (2) dargestellt. Die Größe der Oberfläche wird durch die Kanten (4) und (5) bestimmt. In bevorzugten Ausführungsformen umfasst die mit biologischem Material beschichtete Oberfläche (2) des Festphasenfragments mindestens 5 $mm^2$, mindestens 10 $mm^2$, mindestens 10,25 $mm^2$, mindestens 15 $mm^2$, mindestens 20 $mm^2$, mindestens 25 $mm^2$, mindestens 30 $mm^2$, mindestens 40 $mm^2$, mindestens 45 $mm^2$ oder mindestens 50 $mm^2$.

Kanten des mit biologischem Material beschichteten Festphasenfragments sind in Figur 1 unter den Bezugszeichen (4) und (5) dargestellt. Bevorzugt ist die Länge mindestens einer Kante (4, 5) des Festphasenfragments größer als 1 mm, größer als 1,5 mm, größer als 2 mm, größer als 2,5 mm größer als 3,25 mm, größer als 4 mm, größer als 4,5 mm, größer als 5 mm, größer als 7 mm, größer als 10 mm, größer als 15 mm, größer als 20 mm oder größer als 22 mm. In alternativen Ausführungsformen sind beide Kanten (4) und (5) jeweils größer als die zuvor genannten Längen.

Die Festphasenfragmentdicke ist in Figur 1 unter dem Bezugszeichen 3 dargestellt. Bevorzugt ist die Festphasenfragmentdicke zwischen 0,001 mm und 1,5 mm, zwischen 0,005 mm und 1 mm zwischen 0,01 mm und 0,8 mm, zwischen 0,05 mm und 0,6 mm zwischen 0,06 mm und 0,5 mm, zwischen 0,07 mm und 0,4 mm zwischen 0,08 mm und 0,3 mm und zwischen 0,1 mm und 0,2 mm. Für die Dicke des Festphasenträgers sind die Werte identisch.

Der Ausdruck" in Richtung der beschichteten Oberfläche drücken", wie hierin verwendet, bedeutet, dass der Hebekopf auf der Folie auf der nicht-bestückten Seite direkt unterhalb eines Festphasenfragments angesetzt wird und dieses nach oben drückt. Beim Drücken ändern sich einerseits die Position des Hebekopfs und andererseits die Positionen der Folie und der darauf angebrachten Festphasenfragmente relativ zueinander. Dies kann zum einen dadurch geschehen, dass die Folie starr in ihrer Position verharrt, während der Hebekopf in einer Bewegung von unten nach oben geführt wird. Alternativ kann der Hebekopf starr in einer Position bleiben, während die Folie und die darauf haftenden Festphasenfragmente über den Hebekopf gedrückt werden. In einer dritten Alternative können sich der Hebekopf und die Folie aufeinander zu bewegen, in dem sowohl der Hebekopf als auch die Folie beide ihre Positionen verändern. In bevorzugten Ausführungsformen der vorliegenden Erfindung ist der Hubweg des Hebekopfes in Richtung (oder umgekehrt) 0,1 mm bis 30 mm, 0,3 mm bis 25 mm, 0,5 mm bis 20 mm, 0,8 mm bis 15 mm, 1 mm bis 13 mm, 3 mm bis 10 mm, 4 mm bis 8 mm oder 5,5 mm bis 6,5 mm.

"Nicht beschädigen, zerreißen oder zerstechen", wie hierin verwendet, bezieht sich darauf, dass der Hebekopf nach dem Kontaktieren und Drücken an der Folie keine Schäden hinterlässt. Keine Schäden im Sinne der vorliegenden Offenbarung sind Dehnungen und Verfärbungen der Folie. Schäden im Sinne der vorliegenden Offenbarung sind das Entstehen von Löchern oder Rissen in der Folie, das Entstehen von abstehenden Fasern auf der Folienoberfläche und das Auftreten von Piling, d.h. einer lokalen Anhäufung von Folienmaterial. Daher lenkt der Hebekopf die Folie und die Festphasenfragmente nach Kontaktieren nur aus und hinterlässt ggf. Dehnungen und Verfärbungen auf der Folie.

Der Ausdruck "Rahmen", wie hierin verwendet, bezieht sich auf ein Einspannsystem, auf das eine Folie aufgezogen werden kann. Derartige Systeme sind z.B. als Spannsätze aus der Waferproduktion bekannt. Kommerziell erhältlich sind verwendbare Spannsätze u.a. von Minitron Elektronik GmbH (Ingolstadt, Deutschland), Technovision, Inc. (Nakayama, Japan), Ultron Systems, Inc. (Moorpark, USA) und Thai-Hibex (Pathumthani, Thailand). Der hierin beschriebene Rahmen kann ein Teil der erfindungsgemäßen Zugspannungseinheit sein.

Eine Entnahmeeinheit mit einem Saugnapf, wie sie für die Verwendung im hierin offenbarten Verfahren geeignet ist, wird beispielhaft in EP 1718948 B1 in den Absätzen [0059] bis [0061] und [0063] bis [0066] beschrieben und ist hiermit durch Verweis Gegenstand der vorliegenden Offenbarung.

Ein "Träger", wie hierin verwendet, bezieht sich auf einen Festkörper, an den die erfindungsgemäßen Festphasenfragmente angebracht werden können. Der Träger besitzt mindestens eine ebene Fläche, auf der das oder die Festphasenfragmente aufgebracht werden

können. In bevorzugten Ausführungsformen besteht oder umfasst das Trägermaterial Glas, Polymer oder Silizium. Die Festphasenfragmente und derTrägerwerden durch Kleben, Anschmelzen mindestens einer Oberfläche oder durch ein physikalisches Verbindungssytem (z.B. eine Nut-Feder-Verbindung) zusammengebracht. Bevorzugt ist das Verkleben von Träger und Festphasenfragment. Ferner werden bevorzugt auf einem Träger mindestens ein, mindestens zwei, mindestens drei, mindestens vier, mindestens fünf, mindestens sechs, mindestens sieben, mindestens acht, mindestens neun oder mindestens zehn Festphasenfragmente aufgebracht. In bevorzugten Ausführungsformen kann ein Träger eine Größe von mindestens $5\times25mm^2$, mindestens $15\times25mm^2$, mindestens $25\times25mm^2$, mindestens $40\times25mm^2$ oder mindestens $60\times25mm^2$ besitzen. Beispielsweise kann ein Träger der Größe von mindestens $5\times25mm^2$ mit insgesamt 5 Festphasenfragmenten mit jeweils einer Größe von $5\times5mm^2$; 20 Festphasenfragmenten mit jeweils einer Größe von $2{,}5\times2{,}5mm^2$; oder 125 Festphasenfragmenten mit jeweils einer Größe von $1\times1mm^2$ bestückt werden.

Eine "Vorrichtung zur Herstellung der erfindungsgemäßen Festphasenfragmente", wie hierin offenbart, bezieht sich auf ein oder mehrere Werkteile, die eine Zugspannungseinheit, eine Hebekopfeinheit und eine Entnahmeeinheit, wie hierin beschrieben, umfassen. Die genannten Einheiten können von den anderen Einheiten völlig oder teilweise getrennt vorliegen und ein Herstellungssystem bilden oder alle Einheiten können direkt oder indirekt mit einander verbunden sein.

Der Ausdruck "Antikörper", wie hierin verwendet, bezieht sich auf Proteine aus der Klasse der Globuline, die in Wirbeltieren als Reaktion auf bestimmte Stoffe, so genannte Antigene, gebildet werden. Antikörper sind Bestandteile des Immunsystems. Antikörper werden von einer Klasse weißer Blutzellen, den B-Lymphozyten, produziert. Sie können anhand verschiedener Klassen unterschieden werden, nämlich Immunglobulin A, Immunglobulin D, Immunglobulin E, Immunglobulin G, Immunglobulin M, Immunglobulin W und Immunglobulin Y.

Der Ausdruck "Antigen", wie hierin verwendet, bezieht sich bevorzugt auf Stoffe, an die sich Antikörper und bestimmte Lymphozyten-Rezeptoren spezifisch binden können. Antigene können Proteine, aber auch Glykoproteine, Kohlenhydrate, Lipide oder andere Stoffe sein. Vorliegend sind die Antigene bevorzugt Proteine oder posttranslational modifizierte Proteine.

Generell bedeutet "mindestens 1", wie hierin verwendet, 1, 2, 3, 4, 5, 6, 7, 8, 9 oder mehr, wobei sich die Angabe ggf. auf die Art des genannten Stoffes und nicht die absolute Anzahl der Moleküle bezieht.

Je nach bestimmten Implementierungsanforderungen können verschiedene technische Varianten die Steuereinheit in Hardware und/oder in Software umsetzen. Eine Umsetzung der hier genannten Steuereinheit kann hier als wenigstens eine Steuereinheit erfolgen oder aber durch mehrere Steuereinheiten im Verbund. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente kann als Steuereinheit durch einen Prozessor, einen Computerprozessor (CPU = Central Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikroprozessor (FPGA = Field Programmable Gate Array) gebildet sein.

Obwohl manche Aspekte im Zusammenhang mit den Festphasenfragmenten beschrieben wurden, versteht es sich, dass diese Aspekte auch eine Beschreibung des offenbarten (Herstellungs-)Verfahrens darstellen und umgekehrt.

Zur Vorbereitung für das erfindungsgemäße Verfahren können mit biologischem Material beschichtete Festphasenträger wie folgt hergestellt werden: Biologische Substanzen, wie beispielsweise oben genannt, werden zunächst auf ein Trägermaterial, z.B. papierdünnes (0,15 mm) Glas, wie ein Deckglas, aufgebracht. In der Regel bleibt die biologische Aktivität der zur Beschichtung eingesetzten biologischen Substanzen, die als Substrate bezeichnet werden, unvermindert erhalten. Falls erforderlich kann das biologische Material durch ein Linker-Molekül an den Träger kovalent gebunden werden.

Das mit biologischer Substanz beschichtete Trägermaterial, z.B. ein Deckglas, wird auf einer mit einem Haftkleber versehenen Kunsttoff-Folie, beispielsweise in Form eines Folienrings, befestigt, wobei die biologische Substanz nicht mit der Kunststoff-Folie in Kontakt kommt. Dieser Folienring kann eine Zugspannungseinheit (12) darstellen, die in bevorzugten Ausführungsformen mit einer Steuereinheit (15) in Verbindung steht und durch das Steuersignal SIG2 gesteuert werden kann, z.B. um den Entnahmevorgang auf der offenbarten Vorrichtung elektronisch zu starten (siehe Figur 2).

Das Deckglas wird mit der Substanzschicht, also der Substratseite, nach unten in eine Einsparung oder Schablone einer Vorrichtung gelegt, wobei der nicht beschichtete Deckglasrand aufliegt. Die Schablone dient der definierten Ausrichtung des Deckglases in Position zu Folie und Drehwinkel. Durch Adhäsion wird das Deckglas an die Folie geheftet, wobei die unbeschichtete Seite

des Deckglases auf die im Folienring befindliche Folie trifft.

Das Trägermaterial (Deckglas) wird dann, im Verbund mit den biologischen Substanzen (Substrat), unter Verwendung einer Diamant-armierten Vorrichtung (Fragmentiermaschine oder Fragmentiereinheit) in millimetergroße Fragmente (Festphasenfragmente) unterteilt, ohne dass die Folie zerschnitten wird.

Nach dem Fragmentieren wird das Substrat-bestückte Deckglas auf dem Folienring zwischen einer flexiblen Unterlage und einer Andruckwalze zusätzlich gerollt, um noch nicht gebrochene Substratfragmente zu brechen. Die dabei entstandenen Substratfragmente liegen aber so dicht beieinander, dass deren Kanten aneinander reiben. Um dies zu unterbinden, können die Substratfragmente durch konzentrisches Strecken der sich in dem Folienring befindlichen Folie auf Distanz gebracht werden. Zu diesem Zweck wird die zwischen den beiden konzentrischen Ringen geklemmte Folie nach allen Richtungen gereckt, indem beispielsweise der größere Ring durch Eindrücken in einen identischen Ring ersetzt wird. Man erhält dann sauber geschnittene Fragmente mit genauen Abmessungen und eine exakte Aufteilung der Substratfragmente.

Zum Zwecke des Entnehmens der Festphasenfragmente wird der Folienring auf dem Folienhalter einer Bestückungsmaschine, der in mindestens eine Richtung bewegbar ist, platziert. Der Folienhalter ist im Zentrum durchgängig freigearbeitet.

Die Bestückungsmaschine wird mit Analyseplatten, also beispielsweise bedruckten Objektträgern beladen, die auf einem Transporttablett der Maschine zugeführt werden können. Das Transporttablett kann über einen beweglichen Seitenrand verfügen, der die Objektträger fixiert und so besser positioniert.

Die Bestückungsmaschine ist mit einer Transfereinheit ausgestattet, die aus zwei beweglichen Transportschlitten besteht, die sich auf einer Schiene bewegen. Auf einem Transportschlitten ist ein vakuumunterstützter Saugnapf montiert, der sich angetrieben durch einen Schrittmotor in mindestens zwei, bevorzugt drei Raumrichtung bewegen kann. Der Saugnapf ist pneumatisch unterstützt und drehbar. Außerdem ist der Saugnapf Teil einer Entnahmeeinheit (14), die in bevorzugten Ausführungsformen mit einer Steuereinheit (15) in Verbindung steht und durch das Steuersignal SIG1 gesteuert werden kann, z.B. um die Bewegung des Saugnapfes oder der gesamten Entnahmeeinheit in den Bewegungsrichtungen R1 und R2 elektronisch zu steuern (siehe Figur 2).

Unterhalb des in der Bestückungsmaschine angebrachten Folienringes mit der Substratfragmentbestückten Folie befindet sich eine stempelartige Vorrichtung (Entnahmeeinheit (13)) mit einem Hebekopf, die die Ablösung der Substratfragmente von der Folie unterstützt. Der Hebekopf hebt sich langsam etwa 4-8 mm aus der Ruhestellung hervor (siehe Figur 5), kontaktiert die Folie von unten und bewirkt ein Vorlösen. Das finale Entnehmen wird durch das Ansaugen des Substratfragmentes durch den Saugnapf von oben gesteuert. Der Hebekopf ist Teil einer Hebekopfeinheit (13), die mit einer Steuereinheit (15) in Verbindung stehen kann und durch das Steuersignal SIG3 gesteuert werden kann, z.B. um die Bewegung des Hebekopfs oder der gesamten Hebekopfeinheit (13) in den Bewegungsrichtungen R3 und R4 elektronisch zu steuern (siehe Figur 2).

Ein zweiter sich auf der Transfereinheit der Bestückungsmaschine befindlicher Transportschlitten dient als sogenannter "Kleberschlitten". Er ist mit einer Vorrichtung ausgestattet, die eine bewegliche Klebernadel enthält und über einen Schlauch mit einem Klebstoffreservoir verbunden ist. Vor dem Aufbringen eines Substratfragmentes auf einen Objektträger dosiert oder verteilt die Klebernadel den im Volumen zur Substratfragment-Fläche proportional bemessenen UV-Klebstoff auf die gewählte Bestückungsposition oder Oberfläche des Objektträgers, wobei die Klebernadel mit dem Objektträger in Berührung oder in seine Nähe kommt. Unmittelbar danach wird das Substratfragment auf die Klebstoff-benetzte Stelle des Objektträgers gesetzt. Weitere Substratfragmente werden nun auf gleiche Weise zielgenau auf dem Objektträger platziert. Alternativ kann die Dosierung des Klebstoffes auch kontaktfrei erfolgen. Derartige Systeme sind im Stand der Technik bekannt und werden beispielsweise von der Firma Nordson (Westlake, USA) in Form der "P-Jet CT Jet Ventile", Hochleistungs-Jet-Ventile für die berührungslose Mikrodosierung nieder- bis mittelviskoser Medien, vertrieben.

Die Bestückungsmaschine (Synonym "Vorrichtung zur Herstellung von mit biologischem Material beschichteten Festphasenfragmenten") kann Einheiten enthalten, die der In-Prozess-Kontrolle der durchgeführten Arbeitsschritte und der unmittelbaren Fehlerkorrektur dienen.

Der produzierte Träger, der die Festphasenfragmente umfasst, kann anschließend z.B. verschiedenen Färbetechniken (beispielsweise Hämatoxylin-Eosin-Färbung) zur pathologischen Untersuchung unterzogen werden. Alternativ kann auf dem erfindungsgemäßen Träger ein IIFT-Test, wie beispielsweise von Damoiseaux et al. (Damoiseaux, J. et al. (2009) Journal of Immunological Methods, Journal of Immunological Methods, Band 348, Ausgaben 1-2, Seiten 67-73) beschrieben, durchgeführt werden.

**Fig. 1** zeigt schematisch den Aufbau eines mit biologischem Material beschichteten Festphasenfragments (1).

**Fig. 2** zeigt schematisch den Aufbau einer Vorrichtung zur Herstellung von mit biologischem Material beschichteten Festphasenfragmenten (1), die eine Zugspannungseinheit (12), eine Hebekopfeinheit (13), eine Entnahmeeinheit (14) und eine Steuereinheit (15) umfasst.

**Fig. 3** zeigt diverse Ausführungsformen von Hebeköpfen.

**Fig. 4** zeigt das Ansetzen eines Hebekopfes an eine Folie mit Festphasenfragmenten (1), deren Vorlösen von der Folie und eine Aufsicht auf die Folie nach dem Abnehmen eines Festphasenfragments (1). **(A)** Kontaktieren der Folie mit einem stempelförmigen Hebekopf unterhalb eines Festphasenfragments (1). **(B)** Vorlösen eines Festphasenfragments (1) durch einen stempelförmigen Hebekopf. **(C)** Vorlösen eines Festphasenfragments (1) durch eine Hebekopfeinheit (13), die einen Kugelschreiber umfasst, dessen Spitze als Hebekopf dient. **(D)** Folie mit fragmentiertem Festphasenträger nach dem Abnehmen eines Festphasenfragments (1).

**Fig. 5** zeigt schematisch das Vorlösen eines Festphasenfragments (1) von einer Folie durch einen Hebekopf. **(A)** Haftung eines Festphasenfragments (1) an eine Folie vor Kontaktieren mit einem Hebekopf. Das Festphasenfragment (1) steht mit seiner nicht beschichteten Seite komplett in Kontakt mit der Folie. **(B)** Durch das Erzeugen der Zugspannung an der Folie und das Kontaktieren mit einem Hebekopf, löst sich in der Peripherie der Kontaktstelle das Festphasenfragment (1) von der Folie. Dadurch ist das Festphasenfragment (1) mit seiner nicht beschichteten Seite nur noch partiell in Kontakt mit der Folie.

**Fig. 6** zeigt tabellarisch welche maximalen Festphasenfragmentgrößen durch das Verfahren gemäß EP 1718948 B1 und das erfindungsgemäße Verfahren hergestellt werden können.

**Beispiele**

**Materialien und Geräte:**

- verschiedene Hebeköpfe: Kugelschreibermine, Zylinderkopfschrauben, 3D-Druck-Köpfe, Pass-Stifte, Pipetten-Spitzen;
- Schraubstock zur Aufnahme des Hebekopfes;
- Folie, bespannt im Folienring und bestückt mit Festphasenfragmenten verschiedener Formate

**Beispiel 1: Verfahren zum Herstellen eines mit biologischem Material beschichteten Festphasenfragments mit Hilfe eines Vorlöse-Schritts**

Auf eine Folie (Ultron Systems Sägefolie 1009R) wurde ein Festphasenträger der Größe 76x26x0,15 $mm^3$ aufgezogen und fragmentiert. Der ausgewählte Hebekopf, z.B. der Kopf einer Zylinderkopfschraube (Durchmesser 4,4mm), wurde im Schraubstock möglichst senkrecht eingespannt. Die Folie mit dem fragmentierten Festphasenträger mit dem Format 4,8x4x8x0,15 $mm^3$ wurde per Hand so über den Hebekopf geführt, dass sich der Hebekopf mittig unter einem einzelnen Fragment befindet. Die Folie wurde von Hand langsam, möglichst waagerecht, auf dem Hebekopf abgesetzt (Figur 4A). Durch gleichmäßiges Drücken der Folie auf den Hebekopf wurde ein physikalischer Druck auf das anvisierte Fragment ausgeübt. Dieser Druck wurde solange erhöht, bis sich das Fragment von den benachbarten Fragmenten der Folie gelöst hat (Figur 4B und C). Das Lösen zeigt sich durch ein Erhellen der gelösten Folienfläche. Nach diesem Vorgang haftet das Fragment nur noch an der Fläche, wo zuvor der Hebekopf aufgesetzt wurde. Löst man nun das Fragment von der Folie mit Hilfe eines Saugnapfes, bleibt die hell verfärbte Folienfläche in Größe der Hebekopfkontaktfläche weiterzuerkennen (Figur 4D).

Es zeigt sich, dass es bei der offenbarten Methode nicht notwendig ist, die Folie ein zweites Mal zu recken, was eine erhebliche Einsparung von Arbeitsschritten erlaubt. Das ausgewählte Fragment wird aus der Gesamtmasse herausgehoben und von der Adhäsionskraft der Folie systematisch gelöst. Die umliegenden Fragmente werden zwar gegebenenfalls entsprechend der Auslenkung der Folie auch etwas angehoben. Die Folie stellt sich jedoch nach Beendigung der Ausübung des Druckes zurück und die Fragmente kehren in ihre Ursprungsposition zurück.

**Beispiel 2: Test mit verschiedenen Hebekopfvarianten**

In weiteren Versuchsreihen wurde die Verwendung von verschiedenen Hebekopfvarianten untersucht. Als Hebekopfvarianten wurden Kugelschreiberminen, Zylinderkopfschrauben, 3D-Druck-Köpfe, Pass-Stifte und Pipetten-Spitzen untersucht (Figur 3).

Es wurde gefunden, dass beim quadratischen Format $5{,}0 \times 5{,}0 \times 0{,}15 mm^3$ ab einem bestimmten Durchmesser des Hebekopfes das Fragment gleichmäßig von der Folie abgehoben wird. Unterhalb dieser Größe des Hebekopfs wird das Fragment gekippt von der Folie gelöst und muss auch gekippt von der Folie abgenommen werden (z.B. durch einen Saugnapf).

Die verwendeten Hebeköpfe mit einem Durchmesser von 1,0 bis 1,5 mm sind in der Lage Fragmente auf allen getesteten Formaten von $2{,}5 \times 2{,}5 \times 0{,}15 mm^3$ bis $5{,}0 \times 20{,}0 \times 0{,}15 mm^3$ zu vereinzeln.

Um die Reibung des Hebekopfes auf die Folie, insbesondere bei länglichen Fragmenten, zu verringern hat sich die Verwendung von z.B. Kugelschreiberminen-Spitzen als sinnvoll erwiesen.

**Beispiel 3: Herstellung größerer Festphasenfragmente mit dem erfindungsgemäßen Verfahren**

Die Größe der mit dem erfindungsgemäßen Verfahren maximal herstellbaren Festphasenfragmente

wurde mit solchen verglichen, die mit dem Verfahren gemäß EP 1718948 B1 hergestellt wurden (Figur 6). In beiden Fällen wurde die Folie Ultron Systems 1009R und ein 0,15 mm dicker Festphasenträger verwendet.

Mit dem Verfahren gemäß EP 1718948 B1 können reproduzierbar maximal Fragmente von $3{,}2 \times 3{,}2 mm^2$ Kantenlänge und dementsprechend einer Oberfläche von $10{,}24 mm^2$ hergestellt werden. Mit dem erfindungsgemäßen Verfahren war es möglich Fragmente mit einer Kantenlänge von $25 \times 5{,}0 mm^2$ und einer Oberfläche von $125 mm^2$ herzustellen. Dies entspricht mehr als dem 12,2-fachen der maximalen Oberfläche von Fragmenten, die gemäß dem Verfahren von EP 1718948 B1 hergestellt wurden. Die Herstellung noch größerer Fragmente mittels des erfindungsgemäßen Verfahrens wurde nicht untersucht.

Die Erfindung wird hierin generisch und allgemein beschrieben. Jede der engeren Arten und Untergruppen, die unter die generische Offenbarung fallen, bilden auch einen Teil der Erfindung. Dies schließt die allgemeine Beschreibung der Erfindung mit einer Vorbehalts- oder Negativbeschränkung ein, die jeden Gegenstand von einer (Unter-)Gruppe entfernt, unabhängig davon, ob der ausgeschnittene Gegenstand hier spezifisch zitiert ist oder nicht. Andere Ausführungsformen sind in den folgenden Ansprüchen enthalten.

Ein Fachmann wird leicht erkennen, dass die vorliegende Erfindung gut dazu geeignet ist, die Aufgaben auszuführen und die erwähnten Vorteile sowie die damit verbundenen Ziele zu erreichen. Ferner ist es für den Fachmann ohne weiteres ersichtlich, dass verschiedene Substitutionen und Modifikationen an der hier offenbarten Erfindung vorgenommen werden können, ohne vom Umfang der Erfindung abzuweichen. Die hierin beschriebenen Verfahren, Verwendungen, Behandlungen, Moleküle und Festphasenfragmente sind repräsentativ für bevorzugte Ausführungsformen, die beispielhaft sind und nicht als Beschränkungen des Umfangs der Erfindung gedacht sind. Änderungen darin und andere Verwendungen werden dem Fachmann einfallen, die im Umfang der Erfindung eingeschlossen sind und durch den Umfang der Ansprüche definiert sind. Die Auflistung oder Diskussion eines zuvor veröffentlichten Dokuments in dieser Beschreibung sollte nicht notwendigerweise als Bestätigung dafür verstanden werden, dass das Dokument zum Stand der Technik gehört oder allgemein bekannt ist.

Die hierin veranschaulichend beschriebene Erfindung kann in geeigneter Weise in Abwesenheit von irgendeinem Element oder Beschränkungen, die hier nicht speziell offenbart sind, ausgeführt werden. So werden beispielsweise die Ausdrücke "umfassend", "einschließlich", "enthaltend" usw. umfassend und ohne Einschränkung gelesen. Das Wort "umfassen" oder Variationen, wie "umfasst" oder "umfassend", werden dementsprechend als implizit verstanden, d.h. beispielsweise, dass angegebene Zahlen einbezogen, aber nicht ausgeschlossen werden. Zusätzlich wurden die hierin verwendeten Begriffe und Ausdrücke als Ausdrücke der Beschreibung und nicht der Begrenzung verwendet, und es gibt keine Absicht solche Begriffe und Ausdrücke, um jegliche Äquivalente der gezeigten und beschriebenen Merkmale oder Teile davon zu begrenzen. D.h. dass verschiedene Modifikationen innerhalb des Schutzumfangs der beanspruchten Erfindung möglich sind. Somit sollte verstanden werden, dass, obwohl die vorliegende Erfindung durch beispielhafte Ausführungsformen und optionale Merkmale spezifisch offenbart worden ist, Modifikationen und Variationen der Erfindungen, die hierin offenbart sind, von Fachleuten auf dem Feld verwendet werden können, und dass solche Modifikationen und Variationen als innerhalb des Schutzumfangs dieser Erfindung anzusehen sind.

**Bezugszeichenliste**

| | |
|---|---|
| 1 | Festphasenfragment |
| 2 | Mit biologischem Material beschichtete Oberfläche |
| 3 | Dicke |
| 4, 5 | Kantenlänge |
| 11 | Folie |
| 12 | Zugspannungseinheit |
| 13 | Hebekopfeinheit |
| 14 | Entnahmeeinheit |
| 15 | Steuereinheit |
| SIG1, SIG2, SIG3 | Steuersignal |
| R1, R2, R3, R4 | Bewegungsrichtung |

**Patentansprüche**

**1.** Ein Verfahren zur Herstellung von mit biologischem Material beschichteten Festphasenfragmenten, umfassend:

- Bereitstellen eines mit biologischem Material beschichteten Festphasenträgers, wobei der Festphasenträger fragmentiert ist und die Fragmente an einer Folie haften;
- Erzeugen einer Zugspannung an der mit den Festphasenfragmenten beschichteten Folie;
- Kontaktieren der Folie mit einem Hebekopf, um ein Festphasenfragment von der Folie in der Weise vorzulösen, dass mindestens 20% einer Oberflächenseite des Festphasenfragments von der Folie abgelöst werden, wobei im Kontaktierungsschritt der Hebekopf die Folie in Richtung der beschichteten Oberfläche (2) des Festphasenfragments drückt und wobei das Drücken durch den Hebekopf die Folie nicht beschädigt, zerreißt oder zersticht; und
- finales Ablösen des einen vorgelösten Festphasenfragmentes von der Folie mittels einer

Einheit mit einem Saugnapf, so dass 100% der zuvor gebundenen Oberflächenseite des Festphasenfragmentes von der Folie abgelöst sind.

**2.** Das Verfahren nach Anspruch 1, wobei

(a) die mit biologischem Material beschichtete Oberfläche (2) des Festphasenfragments mindestens 10,25 $mm^2$, bevorzugt mindestens 25 $mm^2$ umfasst; und/oder
(b) das Festphasenfragment ein Glasfragment ist.

**3.** Das Verfahren nach Anspruch 1 oder 2, wobei die Festphasenfragmentdicke (3) zwischen 0,05 mm und 0,3 mm, bevorzugt zwischen 0,1 mm und 0,2 mm ist.

**4.** Das Verfahren nach einem der Ansprüche 1 bis 3, wobei das biologische Material über ein Linkermolekül an den Festphasenträger gebunden ist.

**5.** Das Verfahren nach Anspruch 4, wobei das Linkermolekül

(a) eine Silicium umfassende Verbindung, bevorzugt ein Silylether ist; oder
(b) eine Struktur ausgewählt aus der Gruppe bestehend aus

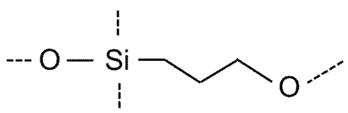

und

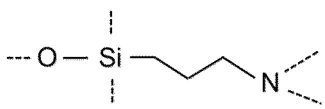

umfasst.

**6.** Das Verfahren nach einem der Ansprüche 1 bis 5, wobei mindestens 70%, bevorzugt mindestens 85% der Oberfläche (2) des Festphasenfragments mit biologischem Material beschichtet ist.

**7.** Das Verfahren nach einem der Ansprüche 1 bis 6, wobei die Zugspannung dadurch erzeugt wird, dass die Folie über einen, bevorzugt kreisförmigen Rahmen gespannt ist.

**8.** Das Verfahren nach einem der Ansprüche 1 bis 7, wobei die Folie eine Klebefolie ist, die

(a) Polyvinylchlorid (PVC); und/oder
(b) Acrylkleber

umfasst.

## Claims

**1.** Method for producing biological material-coated solid-phase fragments, comprising:

- providing a biological material-coated solid-phase support, the solid-phase support being in a fragmented state and the fragments adhering to a film;
- generating a tensile stress on the film coated with the solid-phase fragments;
- contacting the film with a lifting head in order to pre-detach one solid-phase fragment from the film such that at least 20% of a surface side of the solid-phase fragment from the film is detached, wherein, in the contacting step, the lifting head presses the film in the direction of the coated surface (2) of the solid-phase fragment and wherein the pressing by the lifting head does not damage, tear or puncture the film, and
- finally detaching the one pre-detached solid-phase fragment from the film by means of a unit having a suction cup so that 100% of the previously bound surface side of the solid-phase fragment is detached from the film.

**2.** Method according to Claim 1, wherein

(a) the biological material-coated surface (2) of the solid-phase fragment comprises at least 10.25 $mm^2$, preferably at least 25 $mm^2$; and/or
(b) the solid-phase fragment is a glass fragment.

**3.** Method according to Claim 1 or 2, wherein the solid-phase fragment thickness (3) is between 0.05 mm and 0.3 mm, preferably between 0.1 mm and 0.2 mm.

**4.** Method according to any of Claims 1 to 3, wherein the biological material is bound to the solid-phase support via a linker molecule.

**5.** Method according to Claim 4, wherein the linker molecule

(a) is a silicon-comprising compound, preferably a silyl ether; or
(b) comprises a structure selected from the group consisting of

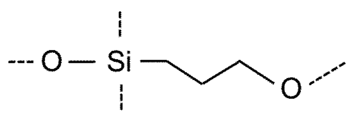

and

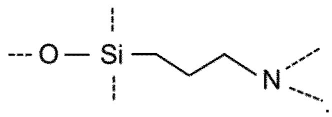

6. Method according to any of Claims 1 to 5, wherein at least 70%, preferably at least 85%, of the surface (2) of the solid-phase fragment is coated with biological material.

7. Method according to any of Claims 1 to 6, wherein the tensile stress is generated by the film being stretched by means of a frame, preferably a circular frame.

8. Method according to any of Claims 1 to 7, wherein the film is an adhesive film comprising

(a) polyvinyl chloride (PVC); and/or
(b) acrylic adhesive.

## Revendications

1. Procédé de fabrication de fragments en phase solide revêtus d'un matériau biologique, comprenant :

- la fourniture d'un support en phase solide revêtu d'un matériau biologique, le support en phase solide étant fragmenté et les fragments adhérant à une feuille ;
- la production d'une contrainte de traction appliquée à la feuille revêtue des fragments en phase solide ;
- la mise en contact de la feuille avec une tête de levage, pour prédétacher de la feuille un fragment en phase solide de façon à détacher de la feuille au moins 20 % d'une face du fragment en phase solide, la tête de levage, dans l'étape de mise en contact, exerçant une pression sur la feuille dans la direction de la face revêtue (2) du fragment en phase solide, la tête de levage ne provoquant aucune dégradation, déchirure ou piqûre de la feuille sous l'effet de la pression appliquée ; et
- le détachement final, à partir de la feuille, du fragment en phase solide prédétaché, à l'aide d'une unité comportant une ventouse, de façon à détacher de la feuille 100 % de la face préalablement liée du fragment en phase solide.

2. Procédé selon la revendication 1, dans lequel

(a) la surface (2), revêtue d'un matériau biologique, du fragment en phase solide a une aire d'au moins 10,25 $mm^2$, de préférence d'au moins 25 $mm^2$ ; et/ou
(b) le fragment en phase solide est un fragment de verre.

3. Procédé selon la revendication 1 ou 2, dans lequel l'épaisseur (3) du fragment en phase solide est comprise entre 0,05 mm et 0,3 mm, de préférence entre 0,1 mm et 0,2 mm.

4. Procédé selon l'une des revendications 1 à 3, dans lequel le matériau biologique est lié au support en phase solide par l'intermédiaire d'une molécule lieur.

5. Procédé selon la revendication 4, dans lequel la molécule lieur comprend

(a) un composé comprenant du silicium, de préférence un silyléther ; ou
(b) une structure choisie dans le groupe consistant en

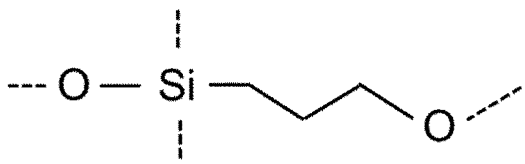

et

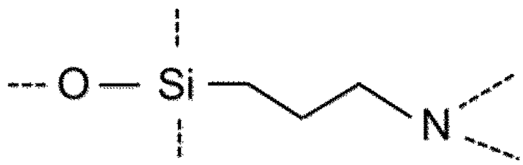

6. Procédé selon l'une des revendications 1 à 5, dans lequel au moins 70 %, de préférence au moins 85 % de la surface (2) du fragment en phase solide sont revêtues du matériau biologique.

7. Procédé selon l'une des revendications 1 à 6, dans lequel la contrainte de traction est produite par serrage de la feuille sur un cadre de préférence circulaire.

8. Procédé selon l'une des revendications 1 à 7, dans lequel la feuille est une feuille adhésive, qui comprend

(a) du poly(chlorure de vinyle) (PVC) ; et/ou
(b) un adhésif acrylique.

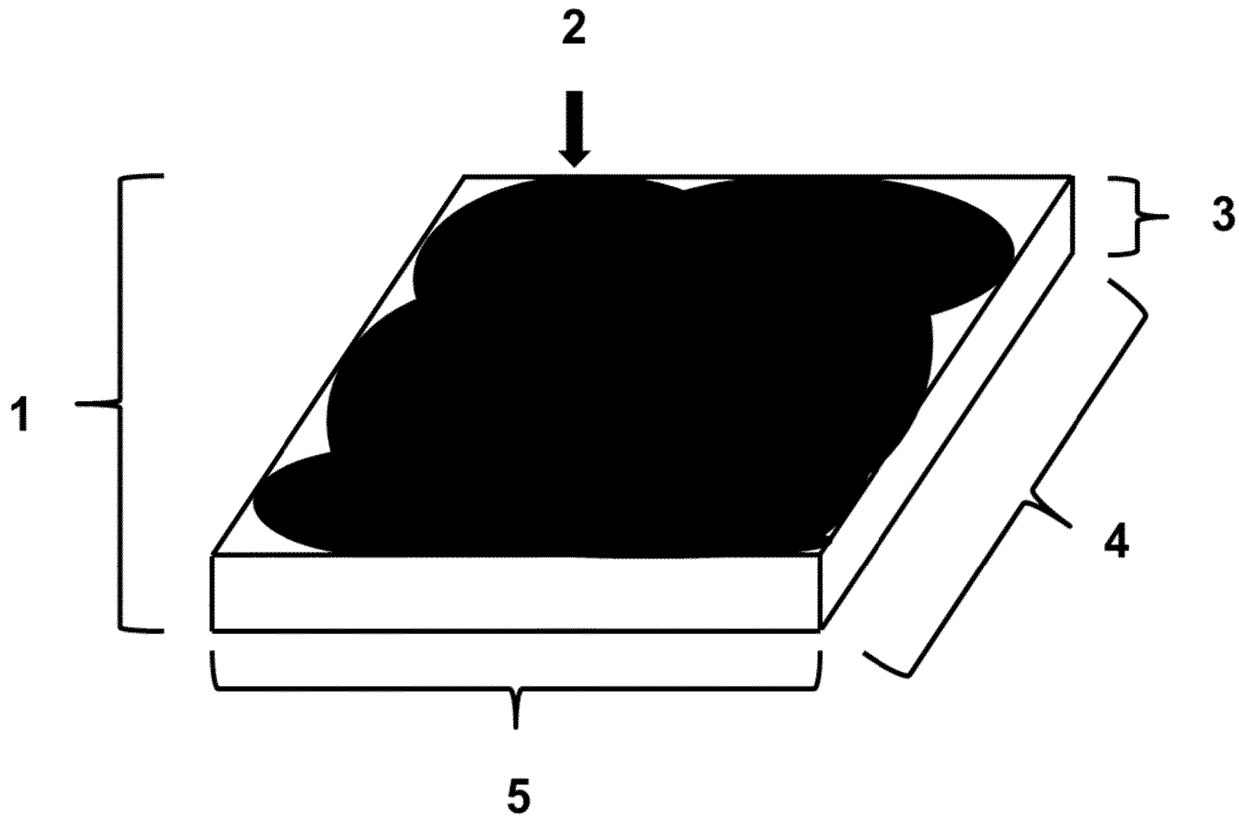


Fig. 1

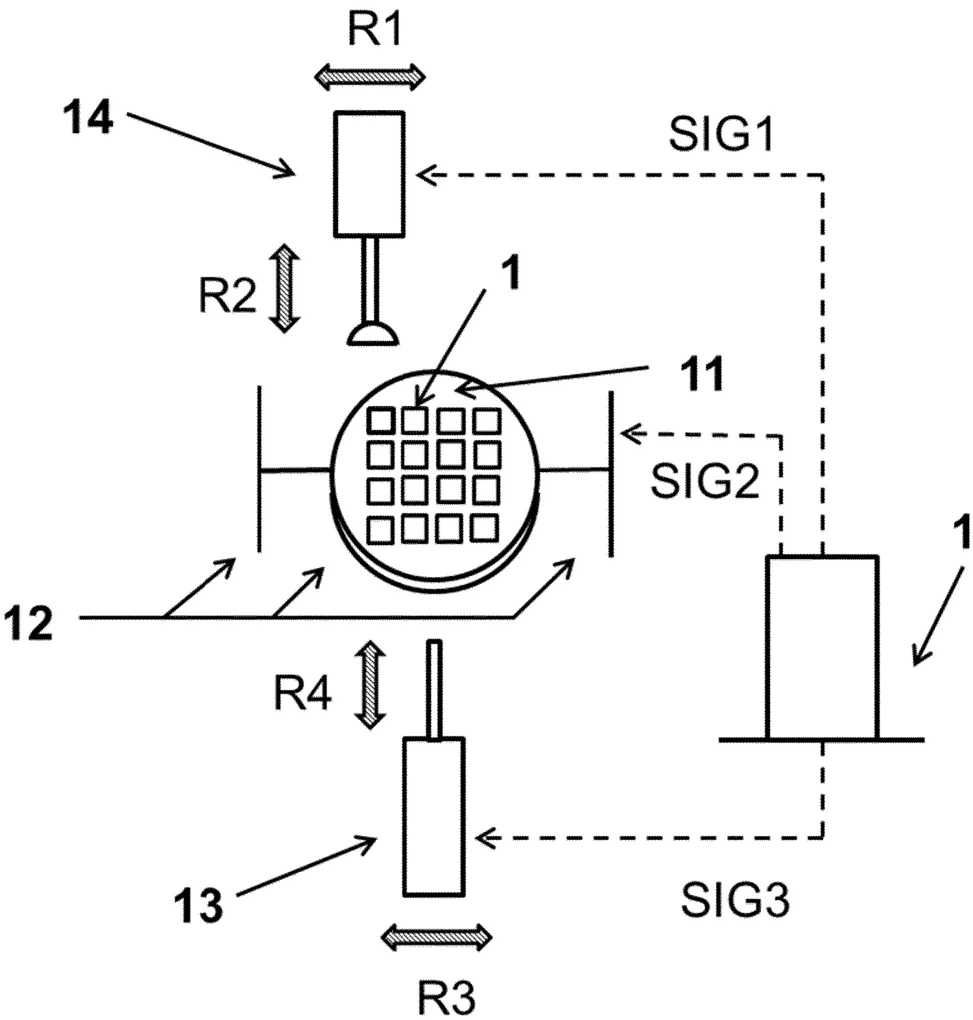


Fig. 2

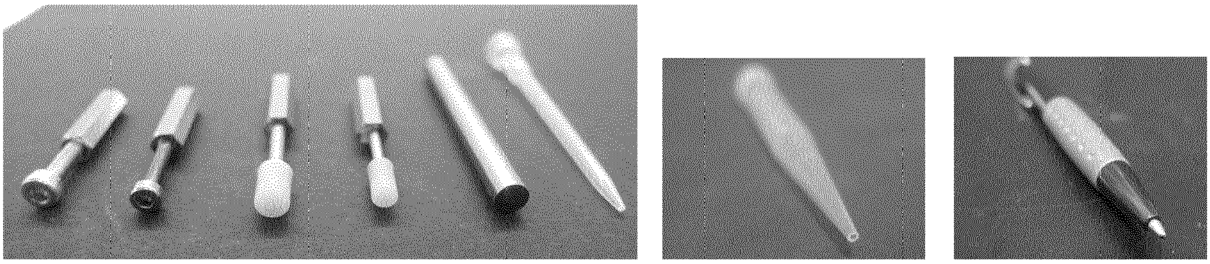

Fig. 3

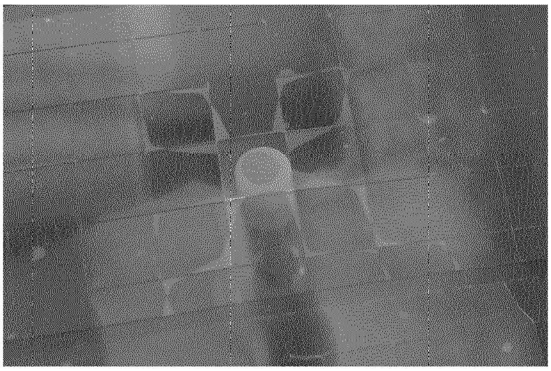

A)

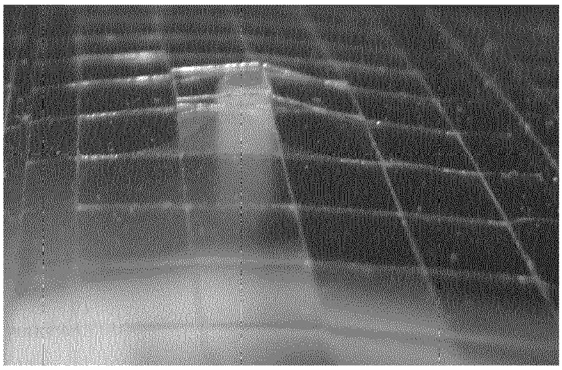

B)

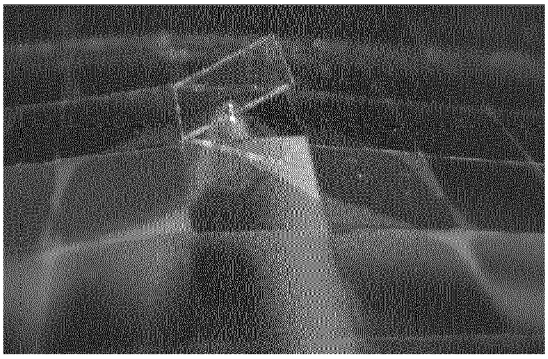

C)

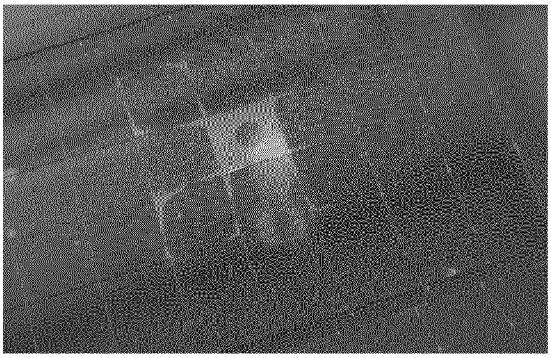

D)

Fig. 4

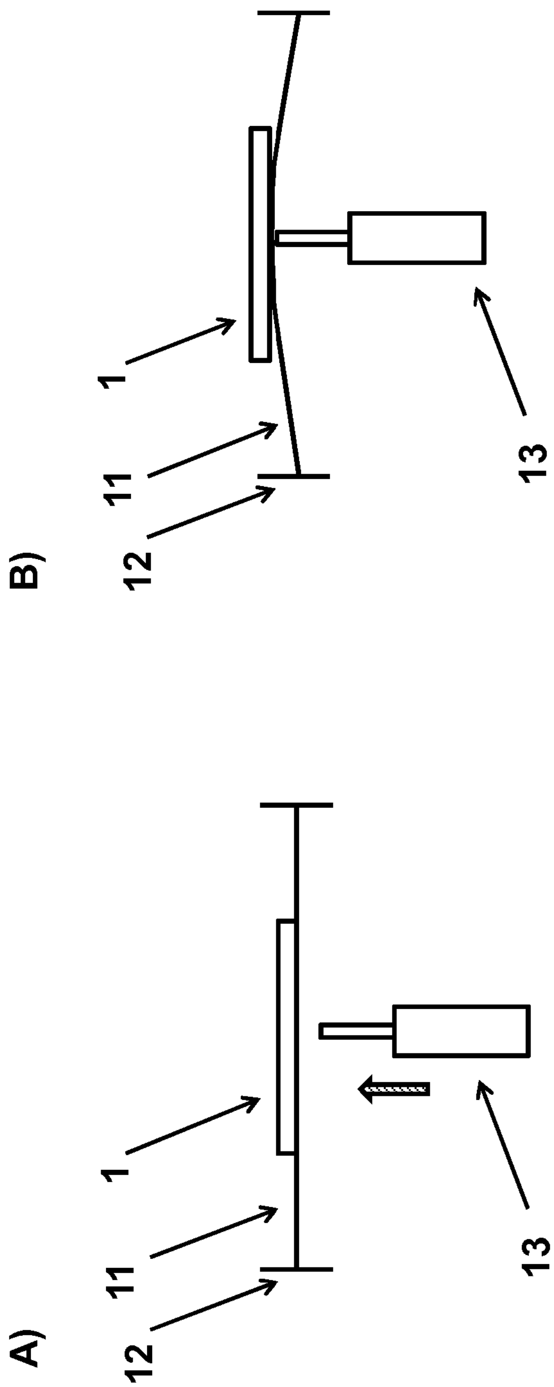


Fig. 5

| Folie | maximale Fragmentgröße | | | |
|---|---|---|---|---|
| | Verfahren (EP 1718948 B1) (maximale Größe ohne Bruch) | | Verfahren (erfindungsgemäß) (maximal getestete Größe) | |
| | Kantenlänge | Oberfläche | Kantenlänge | Oberfläche |
| Ultron Systems 1009R | **3,2mmx3,2mm** | **10,24 mm$^2$** | **25mmx5,0mm** | **125 mm$^2$** |

**Fig. 6**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0117262 B1
- US 4647543 A
- WO 2005073693 A1
- WO 2007140889 A1
- US 2014255978 A1
- EP 1718948 B1


### In der Beschreibung aufgeführte Nicht-Patentliteratur


- **DAMOISEAUX, J. et al.** Journal of Immunological Methods, Journal of Immunological Methods. 2009, vol. 348, 67-73